# EUROPÄISCHE PATENTSCHRIFT

(11) **EP 1 055 927 B1**
(45) Veröffentlichungstag und Bekanntmachung des Hinweises auf die Patenterteilung: **06.10.2004**
(21) Anmeldenummer: 00109435.8
(22) Anmeldetag: 03.05.2000
(51) Int. Cl.: G01N 31/12

(54) **Verfahren und Vorrichtung zum Aufschluss einer wässrigen Lösung zur Kohlenstoffgehaltsbestimmung**
Method and device for the decomposition of a liquid solution to determine the carbon content
Procédé et dispositif pour la décomposition d'une solution liquide afin de déterminer la teneur en carbone

(30) Priorität: 03.05.1999 DE 19920013; 20.05.1999 DE 19923139
(43) Veröffentlichungstag der Anmeldung: 29.11.2000
(73) Patentinhaber: LAR Analytik und Umweltmesstechnik GmbH, 10179 Berlin (DE)
(72) Erfinder: Arts, Werner, Dr., 10825 Berlin (DE); Martens, Berndt, 22941 Bargteheide (DE)
(74) Vertreter: Heinze, Ekkehard, Dipl.-Phys. Dr.

(56) Entgegenhaltungen:
- DE-A- 3 538 778
- DE-A- 4 231 510
- DE-A- 4 231 727
- DE-A- 19 836 215
- DE-C- 4 344 441
- DE-U- 29 814 975
- US-A- 4 352 781
- PATENT ABSTRACTS OF JAPAN vol. 015, no. 368 (P-1253), 17. September 1991 (1991-09-17) & JP 03 144362 A (SHIMADZU CORP), 19. Juni 1991 (1991-06-19)
- PATENT ABSTRACTS OF JAPAN vol. 012, no. 278 (P-738), 1. August 1988 (1988-08-01) & JP 63 058160 A (SHIMADZU CORP), 12. März 1988 (1988-03-12)

## Beschreibung

Die Erfindung betrifft ein Verfahren zum Aufschluß einer wässrigen Lösung zur Bestimmung des Kohlenstoffgehaltes TOC(Total Organic Carbon) sowie eine Vorrichtung zur Durchführung dieses Verfahrens.

Es ist bekannt, wässrige Lösungen, insbesondere Abwasser oder auch Wasser selbst, mit dem Ziel der Bestimmung des Gesamtgehaltes an organischem Kohlenstoff in einem Ofen zu verbrennen und das Verbrennungsgas geeigneten Detektoren zum Nachweis von Verbindungen zuzuführen, deren Erfassung einen Rückschluß auf den Gehalt an organischem Kohlenstoff der wässrigen Lösung erlaubt. Derartige Verbrennungsvorgänge werden üblicherweise im Temperaturbereich zwischen etwa 600 und 850 °C, maximal bis zu 950 °C, ausgeführt. Die hierfür eingesetzten Öfen werden in der Regel mit 220 V Netzspannung betrieben und haben als Heizdrähte Ta-Drähte. Die Verbrennung erfolgt in Gegenwart eines geeigneten Katalysators und wird daher auch als thermisch-katalytischer Aufschluß bezeichnet.

Aus der DE 44 12 778 C1 ist ein Verfahren zur Analyse einer partikelhaltigen wässrigen Probe, insbesondere zur organischen Kohlenstoffbestimmung bekannt, bei dem die Probe in einen ersten Verbrennungsraum eingespritzt, dieser dann durch eine zugeordnete Heizeinrichtung auf etwas 1000 °C aufgeheizt und die Probe hierdurch verdampft und verbrannt wird. Nach vollständiger Verbrennung wird die Heizeinrichtung des ersten Verbrennungsraums abgeschaltet und dieser abgekühlt, und die Verbrennungsgase werden beim Durchlaufen eines einem zweiten, horizontal verlaufenden Verbrennungsraum zugeordneten Katalysators einer Wärmebehandlung im Bereich zwischen 800 °C und 950 °C unterworfen. Der Verbrennungsraum wird bei dieser Anordnung durch ein L-förmiges Quarzglasrohr gebildet. Dessen Nachverbrennungsraum ist mit einem Oxidations-Katalysator, beispielsweise Kupferoxid gefüllt.

In einem Firmenprospekt highTOC wenn es wirklich schwierig wird" der Fa. elementar Analysensysteme GmbH, 63452 Hanau, wird ein TOC-Analysenverfahren beschrieben, bei dem - unter Einsatz des oben bereits erwähnten L-förmigen Verbrennungsrohres - eine matrixfreie katalytische Nachverbrennung bei maximal 1.200 °C nach einer Vorbehandlung zur Trocknung der Probe und Abscheidung von Probenmatrix und Verbrennungsrückständen in einer Patrone erwähnt wird. Angaben zur Art des verwendeten Katalysators enthält diese Druckschrift nicht.

Ein ähnliches Verfahren ist auch aus der ist auch aus der EP 0 887 643 A1 bekannt. Bei diesem Verfahren wird die Probe zunächst von einer Ausgangstemperatur unterhalb der Siedetemperatur des Wassers auf eine Verdampfungstemperatur und in einem zweiten Schritt auf eine wesentlich höhere Verbrennungstemperatur, bevorzugt im Bereich zwischen 800 und 1.000 °C, gebracht. Der Einsatz eines Katalysators wird in dieser Druckschrift nicht spezifiziert; dessen Notwendigkeit ergibt sich aber für den Fachmann aus der Lage des gewählten Temperaturbereiches.

In der DE 43 44 441 C1 wird ein Verfahren zur kontinuierlichen Bestimmung des Gehalts an oxidierbaren Inhaltsstoffen beispielsweise Kohlenstoff in wäßrigen Flüssigkeiten beschrieben, bei dem Flüssigkeits proben in einem Vertikal-Verbrennungs ofen verbrannt werden. Der aus dem Ofen austretende Abgasstrom wird über eine Kreislaufleitung wieder den Ofen zugeführt, wodurch die Zufuhr der Flüssigkeitsprobe bewirkt wird und die erforder liche Einsprühenergie zur Verfügung gestellt wird. Bei der Verbrennung wird ein Katalysator verwendet.

Bei den bekannten katalytischen thermischen Aufschlußverfahren haben sich in der praktischen Handhabung, d.h. im Routinebetrieb von Wasseraufbereitungsanlagen, Klärwerken etc., betriebsorganisatorische Probleme bei der Handhabung der eingesetzten Katalysatoren sowie von salzhaltigen Proben (Industrieabwässern) ergeben. Versäumnisse hierbei können zu Fehlmessungen und in deren Ergebnis zu einer Fehlsteuerung der aufgrund der Meßergebnisse gesteuerten Prozesse oder zu Totalausfällen der Meßvorrichtung führen.

In der erwähnten DE 43 44 441 C1 wird auf ein Verfahren hingewiesen, bei dem die Bestimmung des TOC ohne Katalysator, jedoch bei höheren Temperaturen von 1100 bis 1200°C mit längeren Aufenthaltszeiten in Ofen erfolgt. Dieser Nachteil kann dadurch kompensiert werden, daß die Probe auf ihren Weg durch den Ofen durch Schikanen mehrmals durch die heißeste Ofen zone geleitet wird.

Der Erfindung liegt daher die Aufgabe zugrunde, ein vereinfacht zu handhabendes Aufschlussverfahren sowie eine Vorrichtung zur Durchführung dieses Verfahrens anzugeben.

Diese Aufgabe wird hinsichtlich ihres Verfahrensaspektes durch ein Verfahren mit den Merkmalen des Anspruchs 1 und hinsichtlich ihres Vorrichtungsaspektes durch eine Vorrichtung mit den Merkmalen des Anspruchs 4 gelöst.

Die Erfindung schließt zum einen den wesentlichen Gedanken ein, in Abkehr von der thermisch-katalytischen Art des Aufschlusses einen rein thermischen Aufschluß ohne Anwendung eines Katalysators vorzunehmen. Sie schließt weiter den Gedanken ein, hierzu die Verbrennungstemperatur wesentlich zu erhöhen, und zwar auf Werte oberhalb von 1000 °C und bevorzugt oberhalb von 1200 °C. Letztlich schließt sie den Gedanken ein, hierdurch Salz-Bestandteile der Proben zum Ofenende hindurchzuschmelzen", um sie dort leicht beseitigen zu können.

Der Fortfall des bei bekannten Verfahren benötigten Katalysators bringt neben Kostenvorteilen - die je nach Art des Katalysators von unterschiedlicher Tragweite sind - vor allem den gewünschten betriebsorganisatorischen Vorteil, der sich aus dem Fortfall der Bevorratung mit entsprechendem Katalysatormaterial und der Planung und Ausführung einer periodischen Erneuerung des Materials ableitet. Im Zusammenhang mit der grundsätzlichen Ausschaltung einer diesbezüglich nicht sachgemäßen Handhabung der Anlage erbringt er auch einen Zuverlässigkeitsgewinn. Diese Vorteile wiegen den durch die höhere Verbrennungstemperatur bedingten Nachteil eines geringfügig erhöhten Energieverbrauches bei Betrieb der Anlage bei weitem auf. Durch das Hindurchschmelzen" der Salze können die Wartungsintervalle wesentlich verlängert und die Wartungskosten stark gesenkt werden.

Erfindungsgemäß wird ein Zwei-Schritt-Verfahren angewandt, bei dem die Probe zunächst in einen bevorzugt bei maximal 800 °C gehaltenen ersten Aufschlußbereich und anschließend in den bevorzugt bei mehr als 1200 °C gehaltenen eigentlichen Hochtemperatur-Aufschlußbereich gebracht wird. Der erste Bereich niedrigerer Temperatur dient dabei als Reduktionszone.

Zur Durchführung dieses neuen Verfahrens wird auch eine neue Vorrichtung vorgeschlagen, deren wesentliches Element ein Verbrennungsofen mit dauerhaft hochtemperaturbeständigen Heizelementen, insbesondere aus dem Material Kanthal-Fibrothal®, ist.

Der Verbrennungsofen weist neben einer Hochtemperaturaufschluß-Zone, die bevorzugt auf über 1200 °C gehalten wird, in einer eine gleichzeitige Stickstoffbestimmung ermöglichenden vorteilhaften Ausführung eine weitere, von der erst gekannten getrennte Temperaturzone auf, in der eine wesentlich niedrigere Temperatur, insbesondere von unterhalb 800 °C, gehalten wird. Diese ist oberhalb der Hochtemperaturaufschluß-Zone angeordnet; selbstverständlich wird die konkrete Anordnung auf etwa vorhandene Beschickungsanordnungen abzustimmen sein. Insgesamt hat der Ofen einen vertikalen Aufbau mit ebenfalls vertikal angeordnetem Aufnahmeraum zur Aufnahme eines langgestreckten, im wesentlichen zylinderischen Keramikreaktors, in den von der Ofenoberseite her die zu untersuchende Probe injiziert und das Trägergas eingeleitet werden.

Der Verbrennungsofen weist bevorzugt in den Wandungsbereichen sowie unterhalb und oberhalb der Heizzonen und zwischen diesen eine Keramikfaserisolierung auf.

Der untere Ofenauslaß, wo sich bei der vorgeschlagenen Verfahrensführung die Salze ansammeln, ist größer als üblich dimensioniert.

Der Betrieb erfolgt bevorzugt mit Niederspannung, wobei für die beiden Temperaturzonen, in denen schon aufgrund der unterschiedlichen Temperaturen unterschiedliche Heizleistungen eingesetzt werden, auch unterschiedliche Speisespannungen genutzt werden können.

Besonderheiten weist die bevorzugte Ausführung des Verbrennungsofens auch hinsichtlich der Ofensteuerung auf. Anstelle der für derartige Lasten üblichen Phasenanschnittsteuerung wird eine sogenannte Zweipunkt-Soft-Regelung zu einer "weichen" Lastschaltung im Nulldurchgang unter Steuerung des Impuls-/Pausenverhältnisses eingesetzt.

Vorteile und Zweckmäßigkeiten der Erfindung ergeben sich im übrigen aus den Unteransprüchen sowie der nachfolgenden Beschreibung einer bevorzugten Ausführungsform anhand der Figuren. Von diesen zeigen:
- Fig. 1: eine schematische Darstellung des Aufbaus eines Verbrennungsofens gemäß einer bevorzugten Ausführungsform der Erfindung und
- Fig. 2: ein vereinfachtes Blockschaltbild einer bevorzugten Anordnung zur Steuerung des Verbrennungsofens nach Fig. 1

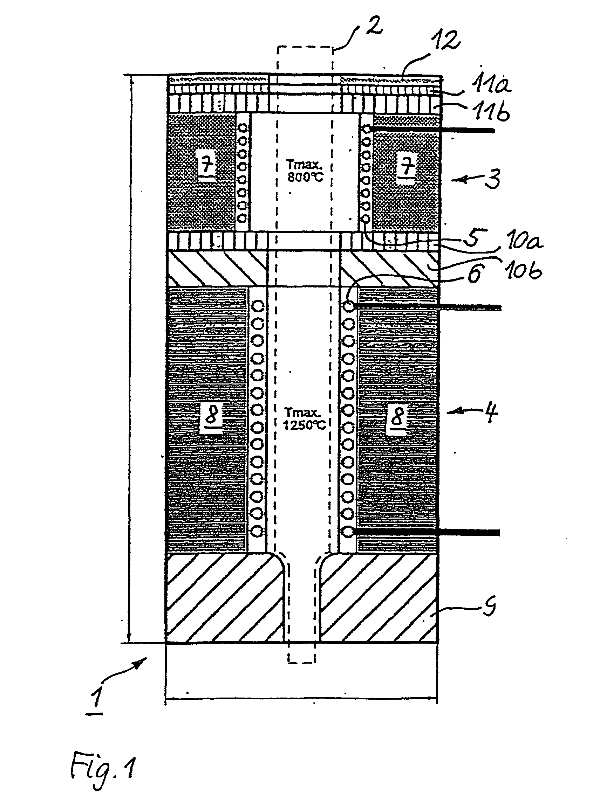

Fig. 1 zeigt einen Probenverbrennungsofen 1 in einer zur Durchführung des erfindungsgemäßen Verfahrens geeigneten Ausführung, in den ein im wesentlichen langgestreckt zylindrisches Keramik-Reaktionsgefäß 1 (in der Figur mit einer gestrichelten Linie umrißartig gezeichnet) einsetzbar ist. Dieses hat am unteren Ende (kaltes Ofenende) einen rohrförmigen Auslaß mit einem Durchmesser im Bereich zwischen 6 und 10 mm, der zur Beseitigung von Salz-Ablagerungen leicht von unten her gereinigt werden kann.

Der Ofen 1 weist eine erste, obere Heizzone 3, in der bei dieser Ausführung eine Maximaltemperatur von 800 °C erreicht werden kann, und eine zweite, untere Heizzone 4 auf, in der eine Maximaltemperatur von 1250 °C erreicht wird. Beide Heizzonen werden über hohlzylindrisch um den entsprechenden Abschnitt des Reaktionsgefäßes 2 angeordnete Heizdrähte 5, 6 aus einer hochtemperaturfesten Speziallegierung, nämlich dem Material Kanthal-Fibrothal®, beheizt. Die obere ebenso wie die untere Heizzone weisen - wegen der unterschiedlichen Maximaltemperaturen unterschiedlich dicke - Kermamikfaser-Isolationen 7 bzw. 8 auf, und auch der Fußbereich, der Bereich 10a, 10b zwischen den Heizzonen und der Bereich 11a, 11b unterhalb eines Aluminiumdeckels 12 sind keramikfaserisoliert. Eine (in der Figur nicht dargestellte) Probenbeschickungs- und Trägergaszuführungseinrichtung ist im Bereich oberhalb des Deckels 12 vorgesehen.

Der in Fig. 1 gezeigte und oben beschriebene Ofenaufbau emöglicht in vorteilhafter Weise die dauerhafte Realisierung speziell der in der zweiten, unteren Heizzone 4 erzeugten hohen Temperaturen, wobei die spezielle Isolierung sowohl zu einem vertretbaren Energieaufwand beiträgt als auch Gefährdungen der Umgebung ausschließt.

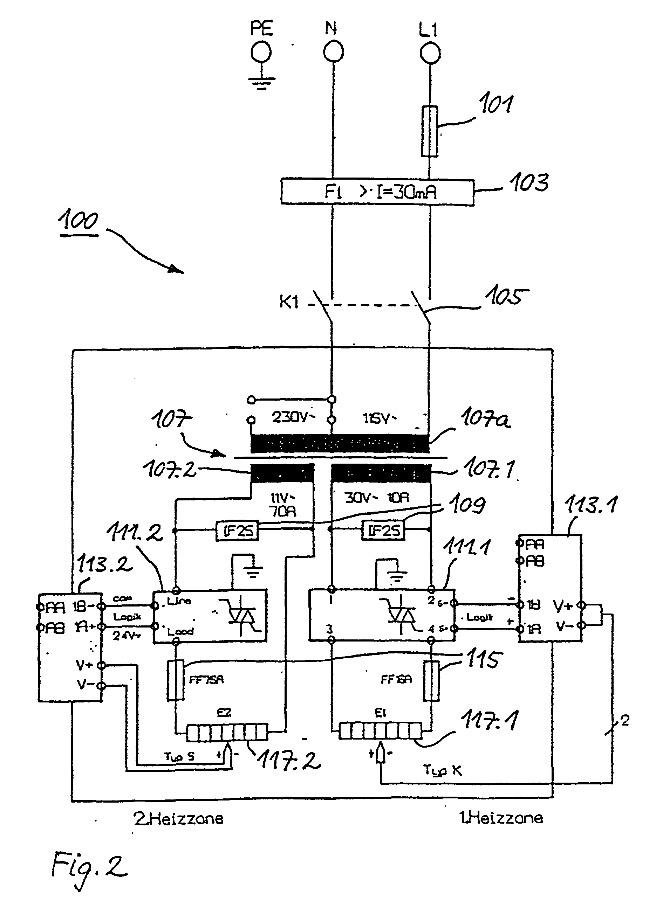

In Fig. 2 ist der Laststromkreis 100 des Verbrennungsofens 1 nach Fig. 1 in einer bevorzugten Ausführungsform dargestellt. Über eine (relative träge) Hauptsicherung 101 und einen FI-Schutzschalter 103 sowie ein Hauptschütz 105 ist eine Primärwicklung 107a eines Spezialtrafos 107 mit Weicheisenverhalten (gekennzeichnet durch eine Induktivität von nur 1,4 T) mit dem Netz verbunden. Der Trafo 107 weist zwei separate Sekundärwicklungen 107.1 für die erste Heizzone (Ziffer 3 in Fig. 1) und 107.2 für die zweite Heizzone (Ziffer 4 in Fig. 1) auf. Obwohl bereits die spezielle Ausführung des Trafos 107 das Auftreten von Störspitzen weitgehend ausschließt, ist für beide Heizzonen jeweils ein Störfilter 109 der Schalteinrichtung, nämlich einem Thyristorschalter 111.1 für die erste Heizzone bzw. 111.2 für die zweite Heizzone, vorgeschaltet. Die Thyristorschalter 111.1, 111.2 werden durch jeweils einen Regler 113.1, 113.2 angesteuert und schalten Heizelementanordnungen 117.1 bzw. 117.2 (entsprechend den Ziffern 5 bzw. 6 in Fig. 1). Zum Schutz der wertvollen Thyristorschalter ist jeweils noch eine superschnelle Sicherung 115 vorgesehen.

Neben dem Spezialtrafo tragen die Regler 113.1, 113.2 durch ein spezielles Zweipunkt-Regelverhalten mit Schaltung ausschließlich im Nulldurchgang zur weitgehenden Vermeidung von Störspitzen bei, wie sie bei "harten" Schaltvorgängen auftreten. Der Trafo und die Regler zusammen realisieren ein für das hier vorgeschlagene Verfahren vorteilhaftes Gesamt-Regelverhalten, das als "Zweipunkt-Soft-Regelung" charakterisiert werden kann. Die Auslegung des Trafos ist im übrigen auf die Eigenschaften der hier eingesetzten Niederspannungs-Heizelemente 117.1, 117.2 aus einer Speziallegierung abgestimmt.

Die Ausführung der Erfindung ist nicht auf das beschriebene Ausführungsbeispiel beschränkt, sondern auch in fachgemäßen Abwandlungen für den konkreten Anwendungsfall möglich.

### Bezugszeichenliste

- 1: Probenverbrennungsofen
- 2: Keramik-Reaktionsgefäß
- 3: erste, obere Heizzone
- 4: zweite, untere Heizzone
- 5, 6: Heizdrähte
- 7, 8: Heizzonenisolierung
- 9: Fußbereich (Isolation)
- 10a, 10b: Zwischenbereich (Isolation)
- 11a, 11b: Deckelisolierung
- 12: Al-Deckel
- 100: Laststromkreis
- 101: Hauptsicherung
- 103: FI-Schutzschalter
- 105: Hauptschütz
- 107: Trafo
- 107a: Primärwicklung
- 107.1, 107.2: Sekundärwicklung
- 109: Störfilter
- 111.1, 111.2: Thyristorschalter
- 113.1, 113.2: Regler
- 115: Sicherung
- 117.1, 117.2: Heizelementanordnung

## Patentansprüche

1. Verfahren zum Aufschluß einer wässrigen Lösung, insbesondere von Abwasser oder Wasser, zur Bestimmung des Gehaltes an organischem Kohlenstoff durch Verbrennung einer Probe, wobei die Verbrennung ohne Gegenwart eines Katalysators bei einer Temperatur oberhalb von 1000°C, insbesondere oberhalb von 1200°C, in einem Vertikalofen erfolgt, der Aufschluß zur Realisierung einer gleichzeitigen Stickstoffbestimmung eine Führung der Probe durch zwei Heizzonen mit unterschiedlicher Temperatur einschließt, von denen eine erste, obere Heizzone niedrigerer Temperatur als Reduktionszone wirkt, und Salzbestandteile im wesentlichen zum kalten unteren Ofenende transportiert werden und sich dort absetzen.

2. Verfahren nach Anspruch 1,
**dadurch gekennzeichnet, daß**
die Temperatur in der Reduktionszone unterhalb von 800 °C liegt.

3. Verfahren nach einem der Ansprüche 1 oder 2,
**dadurch gekennzeichnet, daß**
in Abständen zwischen Meßphasen das kalte Ofenende von Salzablagerungen befreit wird.

4. Vorrichtung zur Durchführung des Verfahrens nach einem der vorangehenden Ansprüche, wobei
ein mit hochtemperaturbeständigen Heizelementen (5, 6), insbesondere aus Kanthal-Fibrotal®, ausgerüsteter Vertikal-Verbrennungsofen (1) vorgesehen ist, der Verbrennungsofen (1) zwei, nämlich eine erste obere Heizzone mit niedrigerer Temperatur und eine zweite, untere Heizzone mit höherer Temperatür, übereinander konzentrisch angeordnete Heizzonen (3, 4) hat, mit eingesetztem langestreckt zylinderischen Reaktionsgefäß (2), insbesondere aus Keramik, welches am kalten Ofenende, d.h. am unteren Ende, einen leicht von unten her reinigenden rohrförmigen Auslaß hat.

5. Vorrichtung nach Anspruch 4,
**dadurch gekennzeichnet, daß**
der Verbrennungsofen (1) für Niederspannung ausgelegt ist.

6. Vorrichtung nach Anspruch 4 oder 5,
**dadurch gekennzeichnet, daß**
der rohrförmige Auslaß einen Innendurchmesser im Bereich zwischen 4 und 15 mm, bevorzugt zwischen 6 und 10 mm, hat

7. Vorrichtung nach einem der Ansprüche 4 bis 6,
**gekennzeichnet durch**
eine Keramikfaserisolierung (7 bis 11b) der Außenfläche und insbesondere zweier Heizzonen (3, 4) des Verbrennungsofens (1) voneinander, wobei die Heizelemente (5, 6) direkt in die Keramikfaserisolierung eingelegt sind.

8. Vorrichtung nach einem der Ansprüche 4 bis 7,
**dadurch gekennzeichnet, daß**
der Verbrennungsofen (1) eine Zweipunkt-Soft-Regelung mit zwei ausschließlich im Nulldurchgang geschalteten Reglern (113.1, 113.2) und einem zugeordneten Trafo, unter Steuerung des Impuls-Pausen-Verhältnisses aufweist.

## Claims

1. A method of decomposing an aqueous solution, in particular of waste water or water, for determining the content of organic carbon by combustion of a sample, with the combustion taking place in the absence of a catalyst at a temperature of above 1000°C, in particular above 1200°C, in a vertical oven,
the decomposition including, for realizing a simultaneous nitrogen determination, a passing of the sample through two heating zones having different temperatures, one of which zones acting as a first, upper heating zone of a lower temperature as a reduction zone; and
salt components essentially being transported to the cold lower oven end and depositing there.

2. The method according to claim 1,
**characterized in that**
the temperature in the reduction zone is below 800°C.

3. The method according to any one of claims 1 or 2,
**characterized in that**
at intervals between measurement phases, the cold oven end is liberated from salt depositions.

4. A device for carrying out the method according to any one of the preceding claims, with
a vertical combustion oven (1) equipped with high temperature-resistant heating elements (5, 6), in particular of Kanthal-Fibrotal® being provided, said combustion oven (1) comprising two heating zones (3, 4) concentrically arranged one above the other, namely a first, upper heating zone having a lower temperature, and a second, lower heating zone having a higher temperature, comprising an inserted elongate cylindrical reaction vessel (2), in particular of ceramics, which has, at the cold oven end, i.e. the lower end, a tubular outlet easy to clean from below.

5. The device according to claim 4,
**characterized in that**
said combustion oven (1) is designed for low voltage.

6. The device according to claim 4 or 5,
**characterized in that**
the tubular outlet has an inner diameter of between 4 and 15 mm, preferably of between 6 and 10 mm.

7. The device according to any one of claims 4 through 6,
**characterized by**
a ceramic fiber insulation (7 through 11b) for insulating the outer surface and, in particular, two heating zones (3, 4) of the combustion oven (1) from each other, with the heating elements (5, 6) being directly embedded in the ceramic fiber insulation.

8. The device according to any one of claims 4 through 7,
**characterized in that**
said combustion oven (1) has an ON/OFF soft control controlled by the pulse-quiescent period-ratio, with two controllers exclusively switched in the zero crossing, and an associated transformer.

## Revendications

1. Procédé de désagrégation d'une solution aqueuse, en particulier d'eaux usées ou d'eau, pour la détermination de la teneur en carbone organique par combustion d'un échantillon, la combustion se réalisant dans un four vertical, en l'absence d'un catalyseur, à une température supérieure à 1000 °C, en particulier supérieure à 1200 °C,
dont la désagrégation comprend, pour réaliser une détermination simultanée de l'azote, un passage de l'échantillon au travers de deux zones de chauffage de température différente dont une première zone de chauffage, de température plus basse, agit en tant que zone de réduction, et où des composants salins sont, pour l'essentiel, acheminés vers l'extrémité inférieure plus froide du four et s'y déposent.

2. Procédé selon la revendication 1, **caractérisé en ce que** la température de la zone de réduction est inférieure à 800 °C.

3. Procédé selon l'une des revendications 1 ou 2, **caractérisé en ce que,** à intervalles entre des phases de mesure, l'extrémité froide du four est dégagée des dépôts de sel.

4. Dispositif pour appliquer le procédé selon l'une quelconque des revendications précédentes, dans lequel
un four de combustion vertical (1) équipé d'éléments de chauffage (5, 6) résistant à de hautes températures, en particulier en Kanthal-Fibrotal ® est prévu, le four de combustion (1) possède deux zones de chauffage (3, 4) concentriques, disposées l'une au-dessus de l'autre, en l'occurrence une première zone de chauffage supérieure de température plus basse et une deuxième zone de chauffage inférieure de température plus haute, avec des récipients de réaction (2) longitudinaux et cylindriques insérés, en particulier en céramique, possédant à l'extrémité froide du four, autrement dit à l'extrémité inférieure, un échappement en forme de tuyau, facile à nettoyer d'en bas.

5. Dispositif selon la revendication 4,
**caractérisé en ce que** le four de combustion (1) est conçu pour une basse tension.

6. Dispositif selon la revendication 4 ou 5,
**caractérisé en ce que** l'échappement en forme de tuyau possède un diamètre intérieur compris entre 4 et 15 mm, de préférence entre 6 et 10 mm.

7. Dispositif selon l'une quelconque des revendications 4 à 6,
**caractérisé par**
une isolation par fibres de céramique (7 à 11b) de la surface extérieure et, en particulier, de deux zones de chauffage (3, 4) du four de combustion (1), les éléments de chauffage (5, 6) étant logés directement dans l'isolation par fibres de céramique.

8. Dispositif selon l'une quelconque des revendications 4 à 7,
**caractérisé en ce que** le four de combustion (1) présente une régulation logicielle deux points avec deux régulateurs (113.1, 113.2) commutés uniquement au passage par zéro et un transformateur adjoint, avec pilotage du rapport impulsion/durée.
